(19) Europäisches Patentamt
European Patent Office
Office européen des brevets

(11) Veröffentlichungsnummer: **0 028 660**
**B1**

(12) **EUROPÄISCHE PATENTSCHRIFT**

(45) Veröffentlichungstag der Patentschrift: **23.10.85**

(21) Anmeldenummer: **79104432.4**

(22) Anmeldetag: **10.11.79**

(51) Int. Cl.[4]: **A 61 K 31/53** // C07D487/04 ,(C07D487/04, 253:00, 231:00)

(54) **Xanthinoxydasehemmer und Verwendung von Benztriazinderivaten.**

(43) Veröffentlichungstag der Anmeldung: **20.05.81 Patentblatt 81/20**

(45) Bekanntmachung des Hinweises auf die Patenterteilung: **23.10.85 Patentblatt 85/43**

(84) Benannte Vertragsstaaten: **AT BE CH DE FR GB IT LU NL SE**

(56) Entgegenhaltungen: **DE - A - 1 470 296**

(73) Patentinhaber: **SIEGFRIED AKTIENGESELLSCHAFT, CH-4800 Zofingen (CH)**

(72) Erfinder: **Thiele, Kurt, Dr., Rebbergstrasse 47d, CH-4800 Zofingen (CH)**
Erfinder: **Gordon Cox, James Stuart, Dr., Eschenweg 7, CH-4800 Zofingen (CH)**
Erfinder: **Fischer, Johanna, Dr., Mattenstrasse 10, CH-6260 Reiden (CH)**
Erfinder: **Jahn, Ulrich, Dr., Kirchmoosstrasse 13, CH-4800 Zofingen (CH)**

(74) Vertreter: **Jaeger, Klaus, Dr.rer.nat. Dipl.-Chem. et al, Jaeger, Grams & Pontani Patentanwälte Bergstrasse 48 1/2, D-8035 München-Gauting (DE)**

**EP 0 028 660 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Xanthinoxydasehemmer sowie die Verwendung von 1,2-Alkylmalonyl-1,2-dihydro-1,2,4-benztriazinderivaten als Wirkstoff zur medikamentellen Behandlung uratischer Diathesen. Unter dem umfassenden Oberbegriff »uratische Diathese« werden dabei die verschiedenen Formen der durch Purinstoffwechselstörungen ausgelösten Hyperurikämie (Gicht) zusammengefaßt.

Trotz eindrucksvoller Fortschritte der Pharmakotherapie bei der Behandlung der verschiedensten Krankheiten ist die Prognose bei Gicht nach wie vor ungünstig. Trotz strengster Diät und eingeschränkter Lebensweise des Kranken sind die mit diesem Leiden verbundenen pathologischen Veränderungen nicht immer zu verhindern. Zur pharmakotherapeutischen Behandlung der uratischen Diathesen stehen nur auffallend wenige Wirkstoffe zur Verfügung, die überdies untereinander keinerlei gemeinsame Strukturmerkmale aufweisen. Genannt seien in diesem Zusammenhang Benzbromaron, Probenecid und Sulfinpyrazon. Diese Substanzen führen über eine Hemmung der Rückresorption von Harnsäure in den Nieren zu einer vermehrten Harnsäure-Ausscheidung. Die erhöhte Harnsäurebildung vermögen diese Wirkstoffe jedoch nicht zu senken, so daß ihre Effektivität beschränkt bleibt und ihr Einsatz durch eine dauernde Überschwemmung der Nieren mit Harnsäure eine Gefahr für dieses Organ bedeutet.

Erst durch die Einführung von Allopurinol in die Behandlung der uratischen Diathese ist die Prognose günstiger geworden. Allopurinol hemmt aufgrund seines der Harnsäure ähnlichen chemischen Aufbaus die Xanthinoxidase und vermag dadurch eine Senkung des Harnsäurespiegels im Organismus herbeizuführen.

Unbefriedigend bei der Verwendung von Allopurinol ist jedoch der nur relativ geringe Plasmaspiegel, der nach oraler Verabreichung der therapeutisch tolerierbaren und üblichen Tagesdosis erzielbar ist. So wird nach einmaliger oraler Gabe von 300 mg/d Allopurinol nur ein Plasmaspiegel von 2 µg/ml erhalten, der nicht einmal ganz dem Dreifachen der 50% inhibitiven Konzentration (IC50) für die Xanthinoxidasehemmung entspricht.

Angesichts dieses Standes der Technik liegt der Erfindung die Aufgabe zugrunde, ein Therapeutikum zu schaffen, das xanthinoxidasehemmend wirkt und den Aufbau relativ hoher Plasmaspiegel ermöglicht.

Zur Lösung dieser Aufgabe schafft die Erfindung eine Verwendung eines Benztriazinderivates der Formel

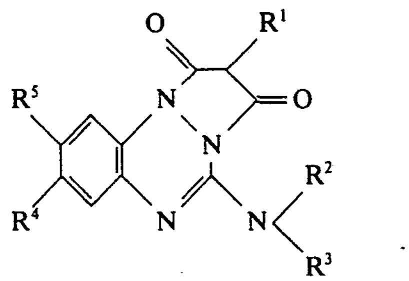

in der

$R^1$ eine Alkylgruppe mit 2 bis 5 Kohlenstoffatomen,
$R^2$ und $R^3$ voneinander Wasserstoff, die Methyl- oder Ethylgruppe und
$R^4$ und $R^5$ unabhängig voneinander Wasserstoff, Chlor oder die Methylgruppe

bedeuten, seiner Salze mit pharmazeutisch unbedenklichen Säuren oder Basen, seinen Hydraten oder Salzhydraten als xanthinoxydasehemmender Wirkstoff zur Herstellung von Fertigarzneimitteln für die medikamentelle Behandlung uratischer Diathesen.

Vorzugsweise bedeuten in der vorstehend wiedergegebenen Formel $R^1$ die n-Propylgruppe oder die n-Butylgruppe, $R^2$ und $R^3$ je die Methylgruppe, $R^4$ Wasserstoff oder die Methylgruppe und $R^5$ die Methylgruppe. Überraschend gute Ergebnisse im Sinne hoher Plasmaspiegel auch bei oraler Gabe der therapeutischen Tagesdosis und zuverlässig wirksamer Xanthinoxydasehemmung werden speziell für das 3-Dimethylamino-7-methyl-1,2-(n-propyl-malonyl)-1,2-dihydro-1,2,4-benztriazin, insbesondere in Form seines Dihydrats, erhalten.

Die unter die vorstehend wiedergegebene Formel fallenden Substanzen sind in der DE-PS 1 470 296 als Antiphlogistika mit analgetischen und antipyretischen Eigenschaften beschrieben. Das 3-Dimethylamino-7-methyl-1,2-(n-propyl-malonyl)-1,2-dihydro-1,2,4-benztriazin, das auch als Pyrazolidinderivat aufgefaßt werden kann und als solches als 5-Dimethylamino-9-methyl-2-propyl-1H-pyrazolo-(1,2 a)-1,2,4-benzotriazin-1,3(2H)-dion zu bezeichnen wäre, ist unter der von der Weltgesundheitsorganisation empfohlenen Kurzbezeichnung (INN) »Azapropazon« bekannt. Azapropazon hat sich seit einer Reihe von Jahren als Antirheumatikum zur Behandlung der verschiedenen Erkrankungen des rheuma

tischen Formenkreises eingeführt. Trotz langjähriger klinischer Erprobung und langjähriger Einführung in die Praxis blieben die xanthinoxydasehemmenden Eigenschaften des Azapropazons sowohl herstellerseitig als auch anwenderseitig völlig unerkannt. Dies überrascht um so mehr als die schlechten Prognosen bei den uratischen Diathesen seit vielen Jahrzehnten ein drückendes Problem sind und sich andererseits zur wirksamen Hemmung der Xanthinoxydase bislang nur ein einziger Wirkstoff, nämlich das Allopurinol, eingeführt hat.

Die überraschende Effektivität der unter die vorstehende Formel fallenden Substanzen als Xanthinoxydasehemmer ist im folgenden anhand eines Vergleiches der Kenndaten für das bekannte Allopurinol und das gemäß der Erfindung verwendete Azapropazon beispielhaft erläutert:

Die Beeinflussung der Xanthinoxydase ist in vitro leicht meßbar, indem verschiedene Konzentrationen einer Prüfsubstanz der Mischung von Hypoxanthin und Xanthinoxydase zugegeben werden und die Bildung von Harnsäure in der gepufferten Lösung während einer Inkubationszeit von 6–8 min durch die Änderung der Absorption bei 290 nm fotometrisch erfaßt wird.

Für die Xanthinoxydasehemmung durch Allopurinol wird eine IC50 von $5 \cdot 10^{-6}$ mol/l entsprechend 0,7 µm/ml bestimmt. Unter gleichen Bedingungen wird für Azapropazon für die Xanthinoxydasehemmung eine IC50 von $4 \cdot 10^{-5}$ mol/l entsprechend 13,5 µg/ml bestimmt. Die therapeutische Tagesdosis, also die maximale Dosis, die im Bereich der Humanmedizin auch bei langfristiger Behandlung unbedenklich verabreicht werden kann, beträgt für Allopurinol 300 mg/d und für Azapropazon 1000 mg/d. Nach einmaliger oraler Gabe der therapeutischen Tagesdosis beträgt der Plasmaspiegel für Allopurinol 2,0 µg/ml, entsprechend dem 2,9fachen der IC50 in vitro, während er für das Azapropazon unter gleichen Bedingungen 80,8 µg/ml, entsprechend dem 6,0fachen der IC50, beträgt. Mit anderen Worten, zur Erzielung einer identischen Wirkung wie sie für das Allopurinol bei oraler Gabe der therapeutischen maximalen Tagesdosis erzielbar ist, braucht vom Azapropazon nur eine Dosis verabreicht zu werden, die unter der halben maximalen antirheumatischen Tagesdosis liegt. Im Vergleich zu Allopurinol, das nur die Xanthinoxydase hemmt, hat Azapropazon zusätzliche gute analgetische und entzündungshemmende Wirkungen und ist daher als polyvalentes Pharmakon zur Behandlung von uratischen Diathesen und als neuer Wirkungstyp anzusehen.

## Patentanspruch

Verwendung eines Benztriazinderivates der Formel

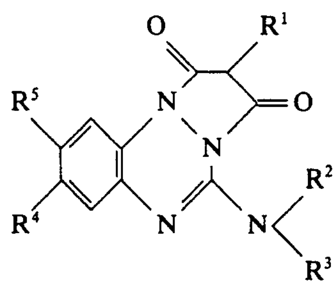

in der

$R^1$ eine Alkylgruppe mit 2 bis 5 Kohlenstoffatomen,
$R^2$ und $R^3$ unabhängig voneinander Wasserstoff, die Methyl- oder Ethylgruppe und
$R^4$ und $R^5$ unabhängig voneinander Wasserstoff, Chlor oder die Methylgruppe

bedeuten, seiner Salze mit pharmazeutisch unbedenklichen Säuren oder Basen, seinen Hydraten oder Salzhydraten als xanthinoxydasehemmender Wirkstoff zur Herstellung von Fertigarzneimitteln für die medikamentelle Behandlung uratischer Diathesen.

**Claim**

Use of derivates of benztriazine of the formula

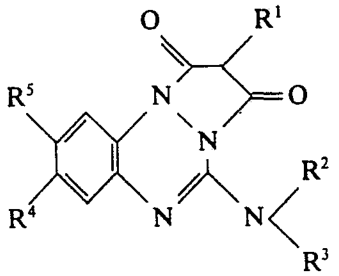

wherein

| | |
|---|---|
| $R^1$ | is an alkyl group comprising 2 to 5 carbon atoms, |
| $R^2$ and $R^3$ | are independently from each other hydrogen, a methyl or ethyl group and |
| $R^4$ and $R^5$ | are independently from each other hydrogen, chlorine or a methyl group, |

salts thereof with acids and bases which are acceptable from a pharmaceutical point of view and the hydrates or salt hydrates thereof as a xanthine oxidase inhibition agent for the manufacturing of medicaments ready for use for the medicamental treatment of uratic diathesis.

**Revendication**

Application d'un dérivé de la benzotriazine répondant à la formule:

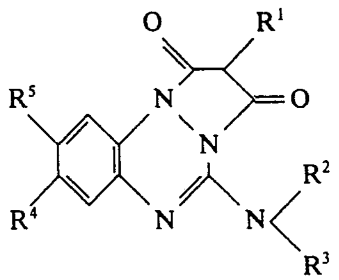

dans laquelle

| | |
|---|---|
| $R^1$ | représente un radical alkyle contenant de 2 à 5 atomes de carbone, |
| $R^2$ et $R^3$ | représentent chacun, indépendamment l'un de l'autre, l'hydrogène, un radical méthyle ou un radical éthyle et |
| $R^4$ et $R^5$ | représentent chacun, indépendamment l'un de l'autre, l'hydrogène, le chlore ou un radical méthyle, |

d'un de ses sels formés avec des acides ou des bases acceptables du point de vue pharmaceutique, de ses hydrates ou de ses sels hydratés, en tant que composé inhibiteur de la xanthine-oxydase pour la préparation de médicaments prêts à l'emploi destinés au traitement médicamenteux de diathèses uratiques.